# EUROPÄISCHE PATENTANMELDUNG

(11) **EP 1 264 583 A1**
(43) Veröffentlichungstag der Anmeldung: **11.12.2002**
(21) Anmeldenummer: 02450121.5
(22) Anmeldetag: 17.05.2002
(51) Int. Cl.: A61F 5/02

(54) **Rückenstütze**

(30) Priorität: 08.06.2001 AT 4612001 U
(71) Anmelder: Pracher, Anton, 3250 Wieselburg (AT); Berger, Felix M. Dr., 3292 Gaming (AT)
(72) Erfinder: Pracher, Anton, 3250 Wieselburg (AT)
(74) Vertreter: Rippel, Andreas, Dipl.-Ing.

(57) **Zusammenfassung**

Bei einer Vorrichtung zur Entlastung des menschlichen Körpers beim Bücken ist mindestens ein federnder Biegestab (3,6) im Beckenbereich befestigt und im Schulterbereich sowie vorzugsweise auch an den Beinen verschiebbar.

Damit wird eine Entlastung des Halte- und Stützapparates erreicht.

## Beschreibung

Die Erfindung bezieht sich auf eine Vorrichtung zur Entlastung des menschlichen Körpers beim Bücken.

Der Arbeitsmediziner steht heute in der Praxis immer mehr vor dem Problem zunehmender Beschwerden am Halte- und Stützapparat. Vor allem die chronischen Schäden an der Wirbelsäule nehmen in bedeutendem Maße zu. Ein wesentlicher Grund dafür ist die Lebensweise der heutigen Zeit, die von Über- und Fehlernährung sowie von Bewegungsmangel bestimmt ist. Dadurch ist der Stoffwechsel und sind sämtliche Regelsysteme im Körper beeinträchtigt, überfrachtet und geschwächt, sodaß oft bereits relativ geringe Belastungen zu Schmerzen am Bewegungsapparat führen.

Eine Entlastung des Halte- und Stützapparates in Kombination mit gesunder Lebensweise und individuell angepaßtem körperlichen Training vermag in hohem Maße Beschwerden und krankheitsbedingte Ausfälle zu verhindern.

Die Erfindung hat es sich zum Ziel gesetzt, eine derartige Entlastung des Halte- und Stützapparates mit Hilfe einer einfachen Vorrichtung zu erreichen. Eine solche erfindungsgemäße Vorrichtung zeichnet sich durch mindestens einen federnden Biegestab aus, der im Beckenbereich befestigt und im Schulterbereich so wie vorzugsweise an den Beinen verschiebbar gelagert ist.

Durch die Verwendung der Vorrichtung nach der Erfindung bei Arbeiten, die in vornübergeneigter Haltung verrichtet werden sollen oder müssen, ist eine wesentliche Verbesserung der Gesundheitssituation des arbeitenden Menschen, eine Erkrankungsvorbeugung für ihn für die kommenden Jahre und damit auch eine deutliche Verringerung der Krankenstandshäufigkeit und der Dauer der krankheitsbedingten Arbeitsunfälle zu erwarten.

Bei einer Ausführungsform einer erfindungsgemäßen Vorrichtung ist die verschiebliche Lagerung an den Beinen oberhalb des Knies vorgesehen.

Es ist im Rahmen der Erfindung aber auch möglich, daß die verschiebliche Lagerung an den Beinen im Knöchelbereich vorgesehen ist.

Um bei einer solchen Lagerung leicht aus der Halterung herausschlüpfen zu können, wird vorgeschlagen, daß die Halterung im Knöchelbereich über eine einseitig offene Spange erfolgt.

Im Rahmen der Erfindung kann die erfindungsgemäße Vorrichtung in einen Overall eingebaut sein. Das Anlegen der Vorrichtung wird damit besonders vereinfacht.

Zur Befestigung der verschiebbaren Lagerung im Schulterbereich ist zweckmäßig eine Tragevorrichtung nach Art eines Rucksackes vorgesehen.

Nachstehend ist die Erfindung anhand eines in den Zeichnungen dargestellten Ausführungsbeispieles näher beschrieben, ohne auf dieses Beispiel beschränkt zu sein. Dabei zeigen:
Fig. 1 in schematischer Ansicht von hinten eine Person mit einer angelegten erfindungsgemäßen Vorrichtung;
Fig. 2 eine Person mit einer angelegten Vorrichtung nach der Erfindung in leicht gebückter Stellung;
Fig. 3 in vergrößertem Maßstab einen Schnitt nach der Linie III-III in Fig. 1;

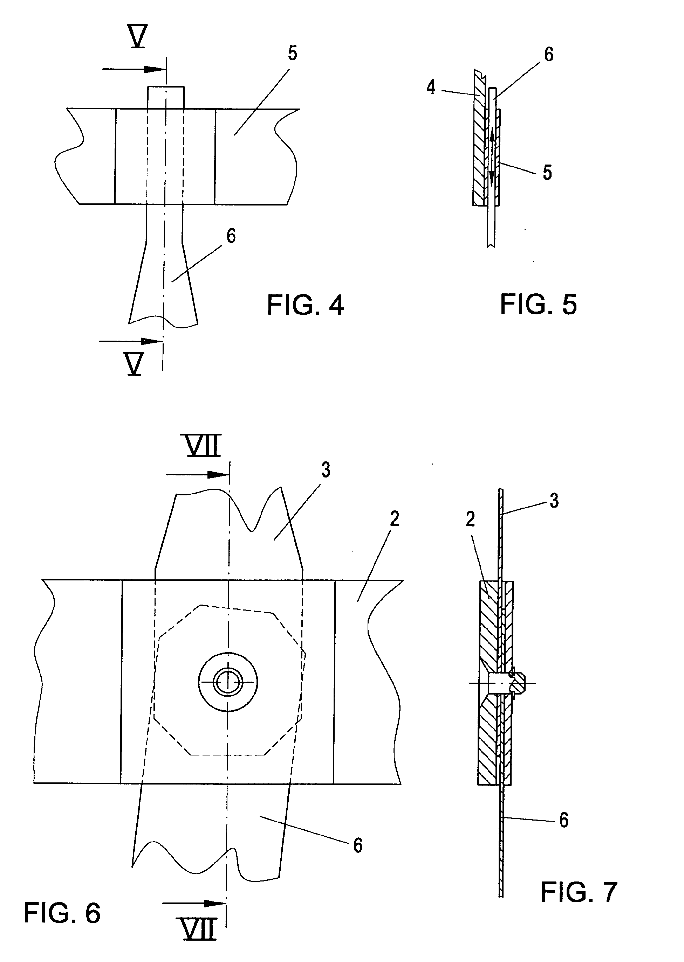

Fig. 4 ebenfalls in vergrößertem Maßstab das Detail IV nach Fig. 1;
Fig. 5 einen Schnitt nach der Linie V-V in Fig. 4;
Fig. 6 in vergrößertem Maßstab das Detail VI in Fig. 1;
Fig. 7 einen Schnitt nach der Linie VII-VII in Fig. 6.

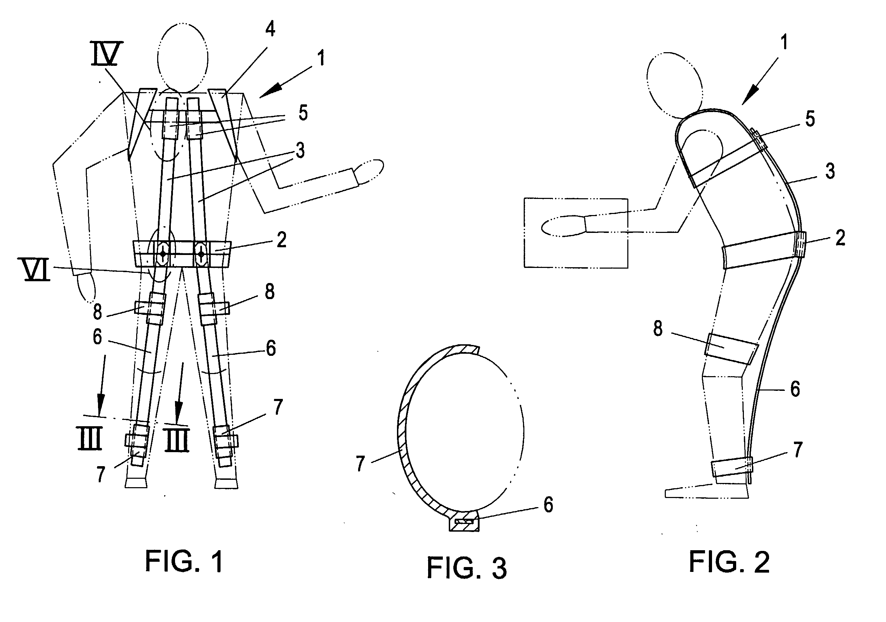

Gemäß den Fig. 1 und 2 trägt eine Person 1 im Beckenbereich einen Gürtel 2, an dem zwei federnde Biegestäbe 3 schwenkbar gelagert sind. Diese Biegestäbe 3 sind im Schulterbereich der Person 1 in Schlaufen 5 eines rucksackartigen Tragegestelles 4 verschiebbar gelagert. Am Gürtel 2 sind überdies zwei federnde Biegestäbe 6 schwenkbar gelagert, die an der anderen Seite in Spangen 7 verschiebbar sind. Diese Spangen 7 umfassen die Beine der Person 1 im Knöchelbereich.

Wie aus Fig. 2 ersichtlich ist, werden beim Bücken der Person 1 insbesondere die Biegestäbe 3, aber auch die Biegestäbe 6 gekrümmt und üben damit eine Kraft aus, die entgegen der Krümmung wirkt. Es können daher auch relativ schwere Lasten mit einem wesentlich geringeren Kraftaufwand gehoben werden. Wird doch das Gewicht des eigenen Oberkörpers von den federnden Stäben bereits bei der Vorneigung im Hüftgelenk getragen. Um dieses Gewicht verringert sich subjektiv die Last für den Hebenden! Die Wirbelsäule bleibt dabei fast gestreckt und beinahe unbelastet.

Diese Vorteile können bei allen auszuführenden Arbeiten und bei allen hebenden Tätigkeiten, sei es in der Landwirtschaft, an Baustellen, bei Lagerarbeiten, an Fließbändern, aber auch bei Arbeiten privat in Haus oder Garten und bei vielen weiteren Gelegenheiten bestens genützt werden.

Durch entsprechende Polsterungen der Schultergurte der Tragevorrichtung sowie der leicht ausschwenkbaren Spangen 7 im Knöchelbereich, um relativ freies Gehen mit dem Gerät zu ermöglichen, und auch der Auflagefläche im Kreuzbein- Lendenbereich, wo sich die Fixierung befindet, ist optimaler Anwendungskomfort bei maximaler Bewegungsfreiheit der Arme gesichert.

Aus den Fig. 1 und 2 ist auch eine Lagerung 8 der Biegestäbe 6 oberhalb des Knies der Person 1 ersichtlich. Diese ebenfalls zweckmäßig als Spange ausgebildete Lagerung 8 kann statt den Spangen 7 im Knöchelbereich verwendet werden.

Im Rahmen der Erfindung sind zahlreiche Abänderungen möglich. So können die Biegestäbe 3 bzw. 6 sowohl aus Kunststoff, zweckmäßig Polyamid, als auch aus Metall bestehen. Die Biegestäbe 3 bzw. 6 können auch teleskopartig ausgebildet sein, sodaß je nach Körpergewicht und Hebelast die Federstärke durch Einstellung mittels Verschieben der Teleskopspangen abgestimmt werden.

Wenn man bedenkt, daß etwa 40% aller krankheitsbedingten Ausfälle auf Schäden am Halte- und Stützapparat zurückzuführen sind, so kann der zu erwartende volkswirtschaftliche Nutzen, der durch die erfindungsgemäße Vorrichtung erreicht werden kann, nicht hoch genug eingeschätzt werden.

## Patentansprüche

1. Vorrichtung zur Entlastung des menschlichen Körpers beim Bücken, **gekennzeichnet durch** mindestens einen federnden Biegestab (3,6), der im Beckenbereich befestigt und im Schulterbereich sowie auch an den Beinen verschiebbar ist.

2. Vorrichtung nach Anspruch 1, **dadurch gekennzeichnet, daß** die verschiebliche Lagerung (8) an den Beinen oberhalb des Knies vorgesehen ist.

3. Vorrichtung nach Anspruch 1, **dadurch gekennzeichnet, daß** die verschiebliche Lagerung (7) an den Beinen im Knöchelbereich vorgesehen ist.

4. Vorrichtung nach Anspruch 3, **dadurch gekennzeichnet, daß** die Halterung im Knöchelbereich über eine einseitig offene Spange (7) erfolgt.

5. Vorrichtung nach einem der Ansprüche 1 bis 4, **dadurch gekennzeichnet, daß** sie in einem Overall eingebaut ist.

6. Vorrichtung nach einem der Ansprüche 1 bis 4, **dadurch gekennzeichnet, daß** zur Befestigung der verschiebbaren Lagerung (5) im Schulterbereich eine Tragevorrichtung (4) nach Art eines Rucksackes vorgesehen ist.
